(19) Europäisches Patentamt
European Patent Office
Office européen des brevets

(11) Veröffentlichungsnummer: **0 004 335**
**A2**

(12) **EUROPÄISCHE PATENTANMELDUNG**

(21) Anmeldenummer: **79100756.0**

(22) Anmeldetag: **14.03.79**

(51) Int. Cl.²: **C 07 D 307/93**, A 61 K 31/34

(30) Priorität: **18.03.78 DE 2811950**

(43) Veröffentlichungstag der Anmeldung: **03.10.79**
**Patentblatt 79/20**

(84) Benannte Vertragsstaaten: **BE CH DE FR GB IT NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Bartmann, Wilhelm, Dr., Am Dachsbau 5, D-6232 Bad Soden am Taunus (DE)**
Erfinder: **Konz, Elmar, Dr., Buchenweg 22, D-6232 Bad Soden am Taunus (DE)**
Erfinder: **Lerch, Ulrich, Dr., Schwalbenweg 19, D-6238 Hofheim am Taunus (DE)**
Erfinder: **Schölkens, Bernward, Dr., Am Fliedergarten 1, D-6233 Kelkheim (Taunus) (DE)**

(54) **Neue Analoga von Prostacyclin, Verfahren und Zwischenprodukte zu ihrer Herstellung und ihre Verwendung als Arzneimittel.**

(57) Die vorliegende Anmeldung betrifft neue Analoga von Prostacyclin, Verfahren und Zwischenprodukte zu ihrer Herstellung und ihre Verwendung als Arzneimittel. Die erfindungsgemäßen Verbindungen zeichnen sich besonders durch ihre hemmende Wirkung auf die Thrombocytenaggregation sowie relaxierende Wirkung auf die Gefäßwand, besonders der Coronararterien, aus.

EP 0 004 335 A2

Neue Analoga von Prostacyclin, Verfahren und Zwischenprodukte zu ihrer Herstellung und ihre Verwendung als Arzneimittel

Prostacyclin oder $PGI_2$, ein kürzlich isolierter Naturstoff aus der Familie der Prostaglandine, zeichnet sich durch seine stark ausgeprägten thrombocytenaggregationshemmenden Eigenschaften aus (The Lancet 1977, 18). Außerdem vermag $PGI_2$ einige Blutgefäße, z.B. Coronararterien zu relaxieren (Prostaglandins 13, 3, 1977), so daß es zur Therapie und Prophylaxe von Thrombosen und Infarkten Verwendung finden könnte.

Gegenstand der vorliegenden Erfindung sind neue Analoga des $PGI_2$ der allgemeinen Formel I

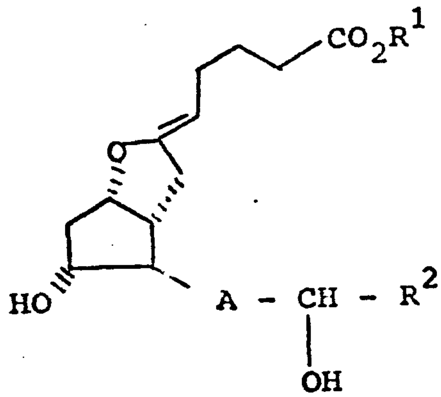

I

in welcher bedeuten

$R^1$ Wasserstoff oder einen geradkettigen oder verzweigten Alkylrest mit einem bis sechs Kohlenstoffatomen, oder einen geradkettigen oder verzweigten ungesättigten aliphatischen Rest mit drei bis sechs Kohlenstoffatomen oder einen cycloaliphatischen Rest mit drei bis sieben Kohlenstoffatomen oder einen araliphatischen Rest mit sieben bis neun Kohlenstoffatomen, oder ein physiologisch verträgliches Metall-, $NH_4$- oder Ammoniumion, das sich von einem primären, sekundären oder tertiären Amin ableitet, oder ein Tetraalkylammoniumion

$R^2$ einen geradkettigen oder verzweigten Alkylrest mit einem bis acht Kohlenstoffatomen, oder einen geradkettigen oder verzweigten ungesättigten aliphatischen Rest mit drei bis acht Kohlenstoffatomen, die ihrerseits substituiert sein können durch

a) einen geradkettigen oder verzweigten Alkoxyrest mit einem bis sechs Kohlenstoffatomen oder einen geradkettigen oder verzweigten Alkenyloxyrest mit drei bis sechs Kohlenstoffatomen, oder

b) einen Cycloalkoxyrest mit drei bis sieben Kohlenstoffatomen

A eine trans 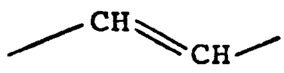 oder $-CH_2-CH_2-$Gruppe.

Gegenstand der Erfindung ist außerdem ein Verfahren zur Herstellung der Prostacyclinderivate der Formel I, dadurch gekennzeichnet, daß man

a) eine Verbindung der Formel II

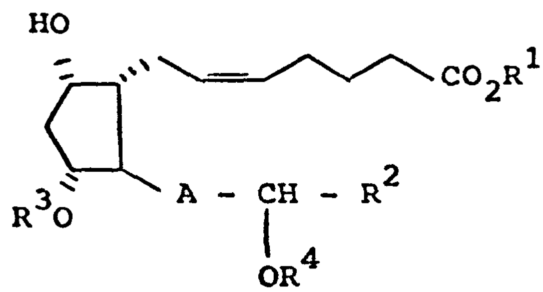

II

worin $R^1$, $R^2$ und A die zur Formel I angegebenen Bedeutungen haben und $R^3$ und $R^4$ gleich oder verschieden sein können und Wasserstoff oder eine leicht abspaltbare Schutzgruppe bedeuten, mit einem geeigneten elektrophilen Reagens umsetzt zu einer Verbindung der Formel III

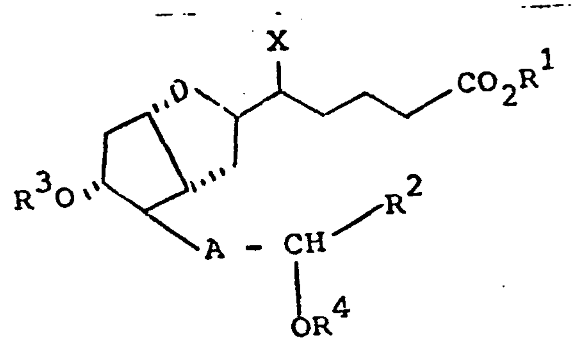

III

worin $R^1$, $R^2$ und A die zur Formel I, $R^3$ und $R^4$ die zur Formel II angegebenen Bedeutungen haben und X Chlor, Brom oder Jod bedeutet,

$a_1$) sofern in einer Verbindung der Formel III $R^3$ und/oder $R^4$ eine Schutzgruppe bedeutet, diese abspaltet, wobei eine Verbindung der Formel III erhalten wird, in welcher $R^1$, $R^2$ und A die zur Formel I und X die zur Formel III angegebenen Bedeutungen haben und $R^3$ und $R^4$ Wasserstoff bedeuten,

b) aus einer Verbindung der Formel III HX abspaltet, wobei eine Verbindung der Formel IV

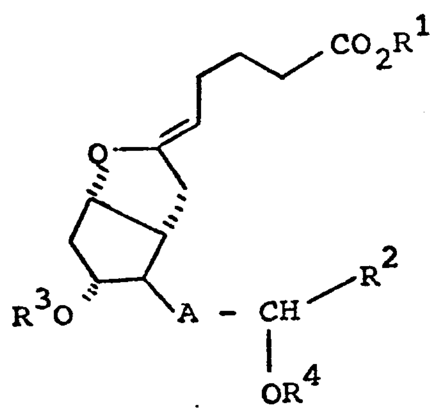

IV

erhalten wird, worin $R^1$, $R^2$ und A die zur Formel I und $R^3$ und $R^4$ die zur Formel II angegebenen Bedeutungen haben,

c) sofern in einer Verbindung der Formel IV $R^3$ und/oder $R^4$ nicht Wasserstoff, sondern eine Schutzgruppe bedeutet, diese unter geeigneten neutralen oder alkalischen Bedingungen abspaltet, wobei eine Verbindung der Formel I gebildet wird, worin $R^1$, $R^2$ und A die zur Formel I angegebenen Bedeutungen haben,

$c_1$) gegebenenfalls aus einer Verbindung der Formel III, sofern $R^3$ und/oder $R^4$ nicht Wasserstoff, sondern eine Schutzgruppe bedeutet, diese gleichzeitig mit HX abspaltet, wobei eine Verbindung der Formel I gebildet wird, worin $R^1$, $R^2$ und A die zur Formel I angegebenen Bedeutungen haben,

d) gegebenenfalls eine Verbindung der Formel I, worin $R^1$ Wasserstoff oder ein Kation bedeutet und A und $R^2$ die zur Formel I angegebenen Bedeutungen haben, zu einer Verbindung der Formel I verestert, worin $R^1$ ein Alkylrest mit der zur Formel I angegebenen Bedeutung ist und A und $R^2$ die zur Formel I angegebenen Bedeutungen haben,

e) gegebenenfalls eine Verbindung der Formel I, worin A und $R^2$ die zur Formel I angegebenen Bedeutungen haben und $R^1$ einen Alkylrest bedeutet, zu einer Verbindung der Formel I verseift, worin A und $R^2$ die zur Formel I angegebenen Bedeutungen haben und $R^1$ Wasserstoff oder ein physiologisch verträgliches Kation bedeutet und

f) gegebenenfalls in einer Verbindung der Formel I, worin A und $R^2$ die zur Formel I angegebene Bedeutung haben und $R^1$ ein physiologisch verträgliches Metall-, $NH_4$- oder Ammoniumion bedeutet, das sich von einem primären, sekundären oder tertiären Amin ableitet, das Kation $R^1$ gegen ein anderes austauscht.

Unter den genannten Substituenten sind die folgenden bevorzugt:

Für $R^1$ Wasserstoff oder ein geradkettiger oder verzweigter Alkylrest mit einem bis sechs Kohlenstoffatomen oder ein cycloaliphatischer Rest mit fünf bis sieben Kohlenstoffatomen oder ein physiologisch verträgliches Metall-, $NH_4$- oder Ammoniumion, das sich von einem primären, sekundären oder tertiären Amin ableitet.

Für $R^2$ insbesondere ein Rest der allgemeinen Formel V

$$-(CH_2)_m-\overset{R^5}{\underset{R^6}{C}}-(CH_2)_n-O-R^7 \qquad V$$

worin $R^5$ und $R^6$ Wasserstoff oder eine Methyl- oder Äthylgruppe bedeuten, wobei $R^5$ und $R^6$ gleich oder verschieden sein können, und $R^7$ einen geradkettigen oder verzweigten Alkylrest mit einem bis fünf Kohlenstoffatomen oder einen Cycloalkylrest mit fünf bis sieben Kohlenstoffatomen bedeutet und m und n gleich oder verschieden sind und die Zahl Null, eins, zwei oder drei darstellen,

wobei im Rest der allgemeinen Formel V die Gesamtanzahl der Kohlenstoffatome zwischen drei und zehn betragen soll.

Unter den Substituenten für $R^2$ sind die im folgenden aufgeführten ganz besonders bevorzugt:

2-Oxapent-1-yl, 3-Oxahex-2-yl, 2-Methyl-3-oxahept-2-yl, 3-Äthyl-4-oxaoct-3-yl, 2-Methyl-3-oxaoct-2-yl, 3-Äthyl-4-oxahept-3-yl, 1-Methyl-1-cyclopentyloxyäth-1-yl, 2,5-Dimethyl-3-oxahex-2-yl, 3-Oxa-2,6,6-trimethylhept-2-yl, 2-Methyl-4-oxahept-2-yl, 3-Äthyl-5-oxahept-3-yl, 1-Cyclohexyloxymethyl-1-methyläthyl, 4-Oxa-2,6,6-trimethylhept-2-yl, 1-Cyclopentyloxymethyl-1-methyl-äthyl, 4-Oxapent-1-yl, 5-Oxahex-2-yl, 6-Methyl-4-oxahept-1-yl, 3-Äthyl-6-oxahept-3-yl, 2,6-Dimethyl-5-oxahept-2-yl, 2-Methyl-5-oxaoxt-2-yl, 4-Cyclopentyloxy-2-methylbut-2-yl, 3,3-Dimethyl-4-oxapentyl, 5-Oxa-2,6,6-trimethylhept-2-yl, 3,3-Dimethyl-4-oxahexyl, 3,3-Dimethyl-4-oxaheptyl, 2,2-Dimethyl-4-oxahexyl, 2,2-Dimethyl-4-oxaheptyl, 2-Methyl-6-oxahept-2-yl, 2-Methyl-6-oxaoct-2-yl, 3-Äthyl-7-oxa-oct-3-yl, 5-Oxaheptyl, 2,7-Dimethyl-6-oxaoct-2-yl, 6-Oxahept-2-yl, 2,2-Dimethyl-5-oxahexyl, 2,2-Dimethyl-5-oxaheptyl, 3,3-Dimethyl-5-oxahexyl, 4,4-Dimethyl-5-oxahexyl, 2,2-Dimethyl-6-oxaheptyl, 3,3-Dimethyl-6-oxaheptyl,

6-Oxaheptyl, 4-Methyl-6-oxaoctyl, 4,4-Dimethyl-6-oxaheptyl.

Für die Herstellung der im erfindungsgemäßen Verfahren als Ausgangsmaterial verwendeten Prostaglandinderivate der allgemeinen Formel II (A: -CH = CH-) kann man analog den Verfahren arbeiten, wie sie z.B. in JACS 91, 5675 (1969), Tetrahedron Lett, 1970, 311 und den Niederländischen Patentschriften 7 206 361 und 7 209 758 beschrieben sind. Die Verbindungen der allgemeinen Formel II (A: $-CH_2-CH_2-$) lassen sich analog dem in der DE-OS 2 355 540 beschriebenen Verfahren herstellen.

Für die Cyclisierung von Verbindungen der Formel II zu Verbindungen der Formel III, worin X vorzugsweise Brom oder Jod bedeutet, eignen sich elektrophile Reagenzien, die mit $\gamma$-Hydroxyolefinen unter Ringschluß zu Tetrahydrofuranderivaten reagieren, wie z.B. Jod, Jodchlorid, $KJ_3$ oder N-Bromimide wie N-Bromsuccinimid, N-Bromcampherimid oder 1,3-Dibrom-5,5-dimethylhydantoin. Die Reaktion wird vorzugsweise in einem inerten Lösungsmittel, wie z.B. Wasser, Methylenchlorid, Chloroform, Diäthyläther, Tetrahydrofuran oder 1,2-Dimethoxyäthan durchgeführt. Es können auch heterogene oder homogene Lösungsmittelgemische verwendet werden. Die Reaktion kann bei Temperaturen zwischen -70 und +30°C durchgeführt werden, gegebenenfalls in Gegenwart eines säurebindenden Mittels wie z.B. Calciumcarbonat, Natriumcarbonat oder Natriumhydrogencarbonat.

Eine bevorzugte Ausführungsform des Verfahrens besteht darin, daß man eine Verbindung der Formel II in Wasser bei 0 bis 10°C mit 1,2 - 3 Äquivalenten $KJ_3$ in Gegenwart von Natriumcarbonat unter Inertgas rührt, mit Natriumthiosulfatlösung das überschüssige $KJ_3$ reduziert und das Cyclisierungsprodukt der Formel III (X = J) mit Chloroform extrahiert. Es kann ohne besondere Reinigung weiter umgesetzt werden.

Sofern in einer Verbindung der Formel III $R^3$ und/oder $R^4$ eine Schutzgruppe bedeutet, kann diese gegebenenfalls abgespalten werden. Dies ist zweckmäßig, wenn es sich um eine Schutzgruppe handelt, die unter Säurekatalyse entfernt werden muß, wie z.B. eine Acetal- oder THP-Gruppe, da Verbindungen der Formel IV säurelabil sind.
Die Schutzgruppen werden unter Säurekatalyse zweckmäßigerweise in einem alkoholischen oder wäßrig/organischen Lösungsmittel abgespalten. Als Säuren eignen sich verdünnte Mineralsäuren oder organische Säuren wie p-Toluolsulfonsäure, Oxalsäure oder Essigsäure. Handelt es sich um eine Acylschutzgruppe, so kann diese im alkalischen Milieu abgespalten werden.

Die Abspaltung von HX aus Verbindungen der Formel III unter Bildung von Verbindungen der Formel IV verläuft unter der Einwirkung von Basen in Gegenwart oder Abwesenheit eines Lösungsmittels.

Als Basen kommen sowohl anorganische wie organische in Betracht, wie z.B. Alkalimetallhydroxyde oder -carbonate, Alkoholate wie z.B. Natriummethylat oder Kaliumtertiärbutylat, Amine wie z.B. Triäthylamin, 4-Dimethylaminopyridin, Dicyclohexyläthylamin oder 1,4-Diazobicyclo/2,2,2/octan, Amidine wie z.B. 1,5-Diazabicyclo/3,4,0/nonen-5 (DBN) oder 1,5-Diazabicyclo/5,4,0/undecen-5 (DBU).

Sofern in einer Verbindung der Formel IV $R^3$ und/oder $R^4$ nicht Wasserstoff, sondern eine Schutzgruppe wie z.B. eine Acylgruppe bedeutet, so kann diese unter milden alkalischen Bedingungen z.B. mit Natrium- oder Kaliumcarbonat in alkoholischer oder alkoholisch-wäßriger Lösung abgespalten werden. Man arbeitet dabei bei-10 bis +30°C.

Man kann auch die Abspaltung von HX und der Schutzgruppen $R^3$ und/oder $R^4$, sofern sie Acylgruppen bedeuten, aus Verbindungen der Formel III gleichzeitig durchführen. Dazu eignet

sich z.B. die Reaktion mit Alkalihydroxyden oder Metallalkoholaten in Wasser oder einem niedrig-Alkylalkohol, wie z.B. Natriummethylat in Methanol. Das Prostacyclinderivat der Formel I kann dadurch direkt gewonnen werden.

Verbindungen der Formel I, worin $R^1$ Wasserstoff oder ein Kation bedeutet und A und $R^2$ die zur Formel I angegebenen Bedeutungen haben, lassen sich zu Verbindungen der Formel I verestern. Wegen der Instabilität der Enolätherstruktur im Prostacyclinmolekül kommen hierfür nur Verfahren infrage, die rasch und unter milden Bedingungen im neutralen oder schwach sauren Milieu, oder vorteilhafterweise im alkalischen Milieu ablaufen. So kann man z.B. ein Prostacyclinderivat der Formel I ($R^1$ = H) bei Temperaturen zwischen -40 und +20°C mit Diazoalkanen der Formel $R^1$-$N_2$ ($R^1$ = Alkyl) verestern, wobei die üblichen Lösungsmittel wie z.B. Diäthyläther, Tetrahydrofuran, Chloroform oder niedermolekulare Alkohole wie Methanol verwendet werden können. Die resultierenden Ester können in einfacher Weise durch Eindampfen des Lösungsmittels isoliert und gegebenenfalls chromatographisch gereinigt werden. Eine bevorzugte Veresterungsmethode besteht darin, daß man das Salz des entsprechenden Prostacyclinderivates I ($R^1$ = Kation) in Gegenwart einer Base wie z.B. eines Metallalkoholates oder Metallcarbonates in einem geeigneten Lösungsmittel mit einem Alkylierungsmittel der Formel $R^1$ - Y, worin Y vorzugsweise Brom, Jod oder einen Sulfonsäurerest bedeutet, umsetzt. Als Metallalkoholate kommen z.B. Natriummethylat, Natriumäthylat oder Kaliumtertiärbutylat in Betracht, als Carbonat eignen sich z.B. Calciumcarbonat oder Natriumhydrogencarbonat. Als geeignete Lösungsmittel kommen Alkohole wie z.B. Methanol oder tert.-Butanol, Äther wie Tetrahydrofuran oder 1,2-Dimethoxyäthan und insbesondere dipolare aprotische Lösungsmittel wie Dimethylformamid, Dimethylsulfoxyd, Acetonitril oder N-Methylpyrrolidon in Betracht.

Verbindungen der Formel I, worin $R^1$ einen Alkylrest bedeutet, können in üblicher Weise in alkalischem Medium zu Verbindungen der Formel I, worin $R^1$ Wasserstoff oder vorzugsweise ein Kation bedeutet, verseift werden, z.B. mit NaOH oder KOH in einem niedermolekularen Alkohol wie Methanol oder Äther wie Dimethoxyäthan oder THF, gegebenenfalls in Gegenwart von Wasser. Vorteilhafterweise benutzt man die äquimolare Menge oder einen sehr geringen Überschuß an Alkalihydroxyd, so daß man das Alkalisalz der Formel I($R^1$ = Alkalimetallion) durch Verdampfen des Lösungsmittels erhält.

Das Alkalikation läßt sich an Ionenaustauschern in üblicher Weise gegen beliebige Kationen austauschen. Dazu läßt man die Lösung des Alkalisalzes eines beanspruchten Prostacyclinderivates durch eine mit einem Kationsaustauscher wie z.B. (R)Amberlite CG - 50 oder (R)Dowex CCR - 2 gefüllte Säule laufen.

Der Kationenaustauscher ist mit dem erwünschten Kation beladen, z.B. mit einem Ammoniumion, das sich von einem primären, sekundären oder tertiären Amin ableitet. Das erwünschte Salz erhält man durch Eindampfen des Eluats.

Die Verbindungen der allgemeinen Formel II und III können als Diastereomerengemisch bezüglich der Stellung der Hydroxylgruppe am Kohlenstoffatom 15 (Prostaglandinnomenklatur), als reine $\alpha$ - oder ß-Isomere oder in Form von optisch aktiven Antipoden für die nachfolgenden Reaktionen eingesetzt werden. Die Trennung von Stereoisomeren bzw. Antipoden kann aber auch nach jeder folgenden Reaktionsstufe erfolgen. Das bedeutet, daß alle beschriebenen Reaktionen mit Diastereomerengemischen, reinen Diastereomeren oder optisch aktiven Antipoden durchgeführt werden können.

Sofern die einzelnen Reaktionsprodukte nicht bereits in genügend reiner Form anfallen, so daß sie für den folgenden Reaktionsschritt eingesetzt werden können, empfiehlt sich

eine Reinigung mittels z.B. Säulen-, Dünnschicht- oder Hochdruckflüssigkeitschromatographie.

Nach den erfindungsgemäßen Verfahren lassen sich außer den in den Beispielen genannten insbesondere auch die folgenden Verbindungen herstellen.

17-Oxa-$PGI_2$-methylester

16,16-Dimethyl-17-oxa-21-homo-$PGI_2$

13,14-Dihydro-17-oxa-16,16,19-trimethyl-$PGI_2$-n-butylester

16,16-Dimethyl-18-oxa-21-homo-$PGI_2$

13,14-Dihydro-16,16-dimethyl-18-oxa-$PGI_2$-propylester

17-Cyclohexyloxy-16,16-dimethyl-18,19,20-trinor-$PGI_2$-methylester

13,14-Dihydro-18-oxa-16,16,20,20,20-pentamethyl-$PGI_2$

19-Oxa-$PGI_2$-äthylester

16,16-Diäthyl-19-oxa-$PGI_2$-methylester

13,14-Dihydro-19-oxa-16,16,20,20-tetramethyl-$PGI_2$-isopropylester

18,18-Dimethyl-19-oxa-$PGI_2$

16,16-Dimethyl-20-oxa-21-homo-$PGI_2$-methylester

16,16-Diäthyl-13,14-dihydro-20-oxa-21,22-dihomo-$PGI_2$

16-Methyl-20-oxa-21-homo-$PGI_2$-äthylester

17,17-Dimethyl-20-oxa-21,22-dihomo-$PGI_2$-methylester

13,14-Dihydro-18,18-dimethyl-20-oxa-21-homo-$PHI_2$

0004335

Die erfindungsgemäßen Verbindungen zeichnen sich insbesondere durch ihre hemmende Wirkung auf die Thrombocytenaggregation aus. Außerdem haben sie relaxierende Wirkung auf die Gefäßwand, besonders die Coronararterien, ferner blutdrucksenkende Eigenschaften. Sie können daher als Arzneimittel angewandt werden.

Die erfindungsgemäßen Verbindungen der Formel I können als freie Säuren, vorzugsweise jedoch in Form ihrer physiologisch unbedenklichen anorganischen oder organischen Salze oder als Ester zur Anwendung kommen.

Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykol oder auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten, sowie örtlich anwendbare Zubereitungen wie Cremes, Emulsionen, Suppositorien oder Aerosole in Frage kommen.

Eine weitere Anwendung der neuen Verbindungen liegt in der Kombination mit anderen Wirkstoffen. Neben anderen geeigneten Substanzen gehören dazu vor allem:

Kreislaufmittel im weitesten Sinne, wie z.B. Herzglycoside wie Digitoxin, Sympathomimetica wie Suprifen, ß-Sympatholytica wie Inderal, Coronardilatatoren wie Chromonar oder Prenylamin, blutdrucksenkende Stoffe wie Reserpin oder

Clonidin, Antiarrhythmica, durchblutungsfördernde Stoffe, Antikoagulantien oder Fibrinolytica, Diuretica wie z.B. Furosemid, Lipidsenker oder Geriatrika und andere stoffwechselwirksamen Präparate, Prostaglandine oder Prostaglandinantagonisten oder Prostaglandin-Biosynthesehemmer, wie z.B. nicht-steroidale Antiphlogistika, Thromboxan-Synthetasehemmer, Psychopharmaka sowie Vitamine.

Die Thrombocyten aggregationshemmende Wirkung der erfindungsgemäßen Verbindungen wurde an der durch Arachidonsäure in Humanblut (in vitro) induzierten Thrombocytenaggregation ermittelt. In der folgenden Tabelle ist die Konzentration an dem jeweiligen Prostacyclin-Derivat angegeben, die eine Aggregation von 50% der vorhandenen Thrombocyten verhindert ($IC_{50}$).

| Verbindung | $IC_{50}$ |
|---|---|
| Beispiel 2 e) | $1 \cdot 10^{-5}$ molar |
| Beispiel 3 a) | $1,2 \cdot 10^{-6}$ molar |
| Beispiel 3 b) | $5,4 \cdot 10^{-7}$ molar |

Zur Hemmung der Thrombocytenaggregation können die erfindungsgemäßen Verbindungen in den folgenden Dosen verabreicht werden:

Einheitsdosis 0,05 - 5 mg, bevorzugt
0,1 - 1 mg

Tagesdosis 0,25 - 25 mg, bevorzugt
0,5 - 5 mg.

Die Verbindungen der Formel III und IV sind neue wertvolle Zwischenprodukte für die Herstellung von Verbindungen der Formel I.

**Beispiel 1:**

a) 5-Brom-16,16-dimethyl-19-oxa-$PGI_1$

Zu 367 mg 16,16-Dimethyl-19-oxa-$PGF_{2\alpha}$-11,15-bis-tetrahydropyranyläther in 7 ml Tetrahydrofuran / Chloroform 1 : 1 gibt man unter Rühren und Argon 132 mg N-Bromsuccinimid. Nach 2 Stunden wird Chloroform und wäßrige Natriumthiosulfatlösung zugegeben und die organische Schicht nach dem Trocknen mit Magnesiumsulfat eingedampft. Den Rückstand erwärmt man unter Argon in 5 ml Eisessig / Wasser / Tetrahydrofuran 3 : 1 : 1 fünf Stunden auf 45° und verdampft danach das Lösungsmittel im Vakuum. Der Rückstand kann an etwa 20 g Silicagel säulenchromatographisch gereinigt werden. Als Elutionsmittel dient Äthylacetat / Eisessig 99 : 1.

b) 16,16-Dimethyl-5-jod-18-oxa-$PGI_1$-methylester

121 mg 16,16-Dimethyl-18-oxa-$PGF_{2\alpha}$-methylester werden in 1,5 ml Diäthyläther gelöst und nach Zugabe von 0,3 g Kaliumhydrogencarbonat in 1 ml $H_2O$ unter Argon auf 0° gekühlt. Innerhalb von 2 Stunden werden 5,4 ml einer 2,5 %igen Lösung von Jod in Äther zugetropft und danach weitere zwei Stunden bei 0° gerührt. Nach der Zugabe von etwa 10 ml Äther wird die organische Schicht zuerst mit Natriumthiosulfatlösung, danach mit Wasser gewaschen, getrocknet und eingedampft. Das Reaktionsprodukt ist genügend rein, kann jedoch durch Chromatographie an

Silicagel (Cyclohexan / Äthylacetat 2 : 8) von geringen Mengen an Verunreinigungen befreit werden.

NMR: δ 5,5 - 5,7 (m, 2H) olef. Protonen,
4,6 (m, 1H),
3,7 (s, 3H) $OCH_3$,
3,3 (s, 2H) O - $\underline{CH_2}$ - $C(CH_3)_2$

c) <u>16,16-Dimethyl-5-jod-17-oxa-$PGI_1$-methylester</u>

79,4 mg 16,16-Dimethyl-17-oxa-$PGF_{2\alpha}$-methylester werden zusammen mit 50,8 mg Jod, 66 mg Kaliumjodid und 42,4 mg Natriumcarbonat in 2 ml Wasser zwei Stunden bei 5 - 10°C unter Inertgas gerührt. Mit Natriumthiosulfatlösung wird anschließend das überschüssige Jod entfärbt und die Reaktionsmischung mit Chloroform extrahiert. Die organische Phase wird mit Wasser gewaschen, getrocknet und eingedampft.

NMR: δ 4,55 (m, 1H),
3,35 (t, 2H) O - $CH_2$,
1,1 (6H) $C(CH_3)_2$
0,9 (t, 3H) $CH_2$ - $\underline{CH_3}$

d) <u>5-Jod-17-oxa-20-dihomo-$PGI_1$-äthylester</u>

Reaktion analog Beispiel 1c aus 17-oxa-20-dihomo-$PGF_{2\alpha}$-äthylester

NMR: δ 5,5 - 5,7 (m, 2H) olef. Protonen,
4,55 (m, 1H),
4,15 (q, 2H) $O\underline{CH_2}$ - $CH_3$,

0004335

3,2 - 3,7 (m, 4H) $OCH_2$

e) 16-Cyclohexyloxy-5-jod-16-methyl-18,19,20-trinor-$PGI_1$-methylester

Reaktion analog Beispiel 1b aus 16-Cyclohexyloxy-16-methyl-18,19,20-trinor-$PGF_{2\alpha}$-methylester

NMR: $\delta$ 5,5 - 5,7 (m, 2H) olef. Protonen,
4,57 (m, 1H),
1,1 und 1,15 (2s, 6H) $C(CH_3)_2$,

f) 5-Jod-16-methyl-19-oxa-13,14-dihydro-$PGI_1$-methylester

Reaktion analog Beispiel 1c aus 16-Methyl-19-oxa-$PGF_{2\alpha}$-methylester

NMR: $\delta$ 4,55 (m, 1H),
3,65 (s, 3H) $CO_2CH_3$,
3,45 (t, 3H) $CH_2O$,
überlagert von 3,3 (s, 3H) $OCH_3$,
0,9 (d, 3H) CH - $CH_3$
kein Signal unterhalb $\delta$ = 5

g) 16,16-Dimethyl-5-jod-18-oxa-13,14-dihydro-20-dihomo-$PGI_1$-methylester

Reaktion analog Beispiel 1c aus 16,16-Dimethyl-18-oxa-13,14-dihydro-20-dihomo-$PGF_{2\alpha}$-methylester

NMR: $\delta$ 4,55 (m, 1H)
3,7 - 4,3 (m, 4H) >CH - O

3,65 (s, 3H) $OCH_3$,

kein Signal unterhalb von $\delta$ = 5

h) <u>16,16-Diäthyl-5-jod-17-oxa-13,14-dihydro-$PGI_1$-methylester</u>

Reaktion analog Beispiel 1c aus 16,16-Diäthyl-17-oxa-13,14-dihydro-$PGF_{2\alpha}$-methylester

NMR: $\delta$ 4,55 (m, 1H),
3,65 (s, 3H) $OCH_3$,
3,3 (t, 2H) O - $\underline{CH_2}$,

i) <u>17,17-Dimethyl-5-jod-19-oxa-$PGI_1$-n-butylester</u>

Reaktion analog Beispiel 1b aus 17,17-Dimethyl-19-oxa-$PFG_{2\alpha}$-n-butylester

NMR: $\delta$ 5,5 - 5,7 (m, 2H) CH = CH,
4,55 (m, 1H),
3,3 und 3,4 (s, 3H und s, 2H) $OCH_3$ und $\underline{OCH_2}-C(CH_3)_2$,
4,05 (t, 2H) $CO_2$ - $CH_2$

k) <u>5-Jod-20-oxa-20-dihomo-$PGI_1$-cyclohexylester</u>

Reaktion analog Beispiel 1c aus 20-Oxa-20-dihomo-$PGF_{2\alpha}$-cyclohexylester

NMR: $\delta$ 5,5 - 5,7 (m, 2H) CH = CH,
4,7 (m, 2H),
3,2 - 3,7 (m, 4H) $OCH_2$

0004335

l) 15-Epi-5-jod-19-oxa-$PGI_1$-benzylester

Reaktion analog Beispiel 1c aus 15-Epi-19-oxa-$PGF_{2\alpha}$-benzylester

NMR: δ 7,35 (s, 5H), arom. Protonen,
4,55 (m, 1H),
3,2 - 3,6 (m, 2H und s, 3H) $OCH_2$ und $OCH_3$

m) 15-Epi-5-jod-17-oxa-20-homo-$PGI_1$-methylester

Reaktion analog Beispiel 1b aus 15-Epi-17-oxa-20-homo-$PGF_{2\alpha}$-methylester

NMR: δ 5,5 (m, 2H) CH = CH,
3,65 (s, 3H) $OCH_3$,
3,2 - 3,6 (m, 4H) $OCH_2$

n) 19,19-Dimethyl-5-jod-17-oxa-$PGI_1$-methylester

Reaktion analog Beispiel 1c aus 19,19-Dimethyl-17-oxa-$PGF_{2\alpha}$-methylester

NMR: δ 5,5 - 5,7 (m, 2H),
4,55 (m, 1H),
3,65 (s, 3H) $OCH_3$,
3,3 - 3,7 (s, und m, 4H) $OCH_2$
überlagert von 3,65 (s, 3H) $OCH_3$,
0,9 (s, 9H) $CH_3$

**Beispiel 2:**

a) 16,16-Dimethyl-19-oxa-$PGI_2$-methylester

Unter einer Argonatmosphäre werden 232 mg 5-Brom-16,16-dimethyl-19-oxa-$PGI_1$ mit 224 mg Kaliumtertiärbutylat in 10 ml tert. Butanol auf 45 - 50$^o$ erwärmt. Nach zweistündigem Rühren wird das Lösungsmittel im Vakuum verdampft, zum Rückstand Eiswasser und kalter Äther gegeben und die wäßrige Phase mit einer kalten Natriumdihydrogenphosphatlösung schnell auf pH 5 gebracht. Die Ätherphase wird sofort mit überschüssigem Diazomethan in kaltem Äther versetzt. Nach 30 Minuten bei -5 bis 0$^o$ reinigt man das Produkt an 15 g Kieselgel, das vorher einige Stunden in einem Gemisch von Äthylacetat / Triäthylamin 95 : 5 aufgeschlämmt war. Man benutzt Äthylacetat / Triäthylamin 98 : 2 als Elutionsmittel

NMR: $\delta$ 5,5 - 5,7 (m, 2H) CH = CH,
4,6 (m, 1H) CH-O,
4,15 (m, 1H) $>C = CH -$,
3,65 (s, 3H) $CO_2CH_3$,
3,45 (t, 2H) $OCH_2$,
überlagert von 3,35 (s, 3H) $OCH_3$,
0,9 und 0,93 (2s, 6H) $C(CH_3)_2$

b) 16,16-Dimethyl-18-oxa-$PGI_2$-methylester

Bei Raumtemperatur rührt man 47,7 mg 16,16-Dimethyl-5-jod-18-oxa-$PGI_1$-methylester mit 0,3 ml 1,5-Diazabicyclo [5,4,0]undecen-5. Nach 30 Minuten ist die Reaktion beendet. Man fügt 3 ml Eiswasser zur Reaktionslösung und extrahiert das Produkt dreimal mit Äther. Die organische Phase wird mit wasserfreiem Natriumsulfat getrocknet, eingedampft und der Rückstand wie oben in Beispiel 2a beschrieben, an Silicagel mit Äthylacetat / Triäthylamin 98 : 2 gereinigt.

NMR: δ 5,5 - 5,7 (m, 2H) CH = CH,
4,6 (m, 1H) CH - O
4,15 (m, 1H) >C = CH -
3,3 - 3,7 (s, 2H; q, 2H; s, 3H) $OCH_2$ und $OCH_3$

c) 16,16-Dimethyl-17-oxa-$PGI_2$-methylester

Reaktion analog Beispiel 2b aus 16,16-Dimethyl-5-jod-17-oxa-$PGI_2$-methylester

NMR: δ 4,6 (m, 1H) CHO,
4,15 (m, 1H) >C = CH -
3,65 (s, 3H) $CO_2CH_3$,
3,35 (t, 2H) $OCH_2$
1,0 u. 1,15 (2s, 6H) $C(CH_3)_2$

d) <u>17-Oxa-20-dihomo-$PGI_2$-äthylester</u>

Reaktion analog Beispiel 2b aus 5-Jod-17-oxa-20-dihomo-$PGI_1$-äthylester

NMR: δ 5,5 - 5,7 (m, 2H) CH = CH,
4,6 (m, 1H) CH - O,
3,7 - 4,3 (m, 5H) $CO_2CH_2$, >C = CH, CH - O (2)

e) <u>16-Cyclohexyloxy-16-methyl-18,19-20-trinor-$PGI_2$-methylester</u>

68,3 mg 16-Cyclohexyloxy-5-jod-16-methyl-18,19,20-trinor-$PGI_1$-Methylester erwärmt man unter Argon mit 81 mg wasserfreiem Natriummethylat in 1 ml absolutem Methanol auf 40°C. Nach zwei Stunden dampft man die Reaktionsmischung im Vakuum ein und verteilt den Rückstand zwischen Eiswasser und kaltem Äther. Die Ätherphase wird getrocknet und eingedampft und das rohe Prostacyclinderivat, wie in Beispiel 2 a beschrieben, chromatographisch gereinigt.

NMR: δ 5,5 - 5,7 (m, 2H) CH = CH,
4,6 (m, 1H) CH - O,
4,15 (m, 1H) >C = CH -,
3,95 (m, 1H) CH - O,
3,65 (s, 3H) $CO_2CH_3$,
1,1 und 1,15 (2s, 6H) $C(CH_3)_2$

f) <u>16-Methyl-19-oxa-13,14-dihydro-$PGI_2$-methylester</u>

Reaktion analog Beispiel 2b aus 5-Jod-16-methyl-19-oxa-13,14-dihydro-$PGI_1$-methylester

NMR: δ 4,6 (m, 1H),
4,15 (m, 1H) >C = CH -,
3,65 (s, 3H) $CO_2CH_3$,
3,45 (t, 3H) $CH_2O$,
3,3 (s, 3H) $OCH_3$
0,9 (d, 3H) >CH - $CH_3$

g) <u>16,16-Dimethyl-18-oxa-13,14-dihydro-20-dihomo-$PGI_2$-methylester</u>

Reaktion analog Beispiel 2e aus 16,16-Dimethyl-5-jod-18-oxa-13,14-dihydro-20-dihomo-$PGI_1$-methylester

NMR: δ 4,6 (m, 1H),
4,15 (m, 1H) >C = CH - ,
3,65 (s, 3H) $CO_2CH_3$,
3,3 - 3,7 (s und q, 4H) $OCH_2$

h) <u>16,16-Diäthyl-17-oxa-13,14-dihydro-$PGI_2$-methylester</u>

Reaktion analog Beispiel 2b aus 16,16-Diäthyl-5-jod-17-oxa-13,14-dihydro-$PGI_1$-methylester

NMR: δ 4,6 (m, 1H),
4,15 (m, 1H) >C = CH-,
3,65 (s, 3H) $CO_2CH_3$,
3,3 (t, 2H) $OCH_2$

i) <u>17,17-Dimethyl-19-oxa-$PGI_2$-n-butylester</u>

Reaktion analog Beispiel 2b aus 17,17-Dimethyl-5-jod-19-oxa-$PGI_1$-n-butylester

NMR: $\delta$ 5,5 - 5,7 (m, 2H) CH = CH,
4,6 (m, 1H) CHO
3,7 - 4,3 (5H, m) >C = CH und 2 CH - O und $CO_2CH_2$
3,3 und 3,4 (s, 3H und s, 2H) $OCH_3$ und $OCH_2$

k) <u>20-Oxa-20-dihomo-$PGI_2$-cyclohexylester</u>

Reaktion analog Beispiel 2b aus 5-Jod-20-oxa-20-dihomo-$PGI_1$-cyclohexylester

NMR: $\delta$ 5,5 - 5,7 (m, 2H) CH = CH,
4,7 (m, 2H) CH - O und $CO_2CH$,
3,2 - 3,6 (m, 4H) $OCH_2$

l) <u>15-Epi-19-oxa-$PGI_2$-benzylester</u>

Reaktion analog Beispiel 2b aus 15-Epi-5-jod-19-oxa-$PGI_1$-benzylester

NMR: $\delta$ 7,35 (s, 5H) arom. Protonen,
4,6 (m, 1H) CHO,
3,45 (t, 3H) $CH_2O$,
3,3 (s, 3H) $OCH_3$

m) 15-Epi-17-oxa-20-homo-$PGI_2$-methylester

Reaktion analog Beispiel 2b aus 15-Epi-5-jod-17-oxa-20-homo-$PGI_1$-methylester

NMR: δ 5,5 - 5,7 (m, 2H) CH = CH,
4,6 (m, 1H) CHO,
3,65 (s, 3H) $CO_2CH_3$,
3,2 - 3,6 (m, 4H) $OCH_2$

n) 19,19-Dimethyl-17-oxa-$PGI_2$-methylester

Reaktion analog Beispiel 2b aus 19,19-Dimethyl-5-jod-17-oxa-$PGI_1$-methylester

NMR: δ 5,5 - 5,7 (m, 2H),
4,6 (m, 1H) CH - O
3,2 - 4,2 (m, 10H) $OCH_2$, $OCH_3$, CH - OH und >C = CH -,
0,9 (s, 9H) $CH_3$

Beispiel 3:

a) Kaliumsalz des 16,16-Dimethyl-19-oxa-$PGI_2$

192 mg reiner 16,16-Dimethyl-19-oxa-$PGI_2$-methylester, 1,1 ml 0,5 M Kaliumhydroxydlösung und 2 ml Methanol läßt man unter Inertgas 24 Stunden bei Raumtemperatur stehen. Das Methanol wird im Vakuum abgezogen und die wäßrige Lösung des Kaliumsalzes gefriergetrocknet. Man erhält das Kaliumsalz des Prostacyclinderivates als farbloses Pulver.

b) Natriumsalz des 16,16-Dimethyl-18-oxa-$PGI_2$

Die Verseifung des 16,16-Dimethyl-18-oxa-$PGI_2$-methylesters erfolgt analog zu der in Beispiel 3a beschriebenen Methode mit einer Natriumhydroxydlösung.

c) Triäthylammoniumsalz des 16,16-Dimethyl-18-oxa-$PGI_2$

Eine wäßrige Lösung von 50 mg des Natriumsalzes von 16,16-Dimethyl-18-oxa-$PGI_2$ wird auf eine Säule mit 15 g Amberlite CG - 50 (Triäthylammonium-Form) gegeben. Man eluiert mit einer 2%igen wäßrigen Lösung von Triäthylammoniumcarbonat. Durch Gefriertrocknen des Eluates erhält man das Produkt.

In Analogie zu den Beispielen 3 a bis 3 c lassen sich aus den Verbindungen der Beispiele 2 c bis 2 n durch alkalische Esterverseifung und ggf. Chromatographie an Ionenaustauschern die entsprechenden Alkali- oder Ammoniumsalze herstellen.

PATENTANSPRÜCHE:

1. Verbindungen der Formel I

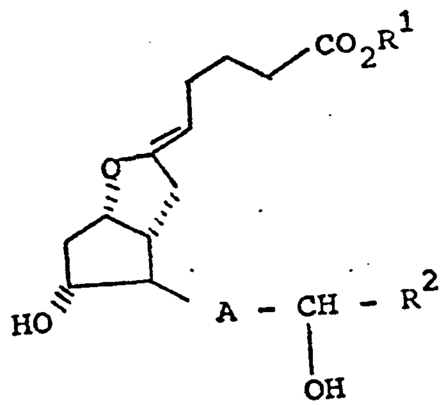

I

worin bedeuten:

$R^1$ Wasserstoff oder einen geradkettigen oder verzweigten Alkylrest mit einem bis sechs Kohlenstoffatomen, oder einen geradkettigen oder verzweigten ungesättigten aliphatischen Rest mit drei bis sechs Kohlenstoffatomen oder einen cycloaliphatischen Rest mit drei bis sieben Kohlenstoffatomen oder einen araliphatischen Rest mit sieben bis neun Kohlenstoffatomen, oder ein physiologisch verträgliches Metall-, $NH_4$- oder Ammoniumion, das sich von einem primären, sekundären oder tertiären Amin ableitet oder ein Tetraalkylammoniumion,

$R^2$ einen geradkettigen oder verzweigten Alkylrest mit einem bis acht Kohlenstoffatomen, oder einen geradkettigen oder verzweigten ungesättigten aliphatischen Rest mit drei bis acht Kohlenstoffatomen, die ihrerseits substituiert sein können durch

a) einen geradkettigen oder verzweigten Alkoxyrest mit einem bis sechs Kohlenstoffatomen oder einen geradkettigen oder verzweigten Alkenyloxyrest mit drei bis sechs Kohlenstoffatomen, oder

b) einen Cycloalkoxyrest mit drei bis sieben Kohlenstoffatomen

A eine trans 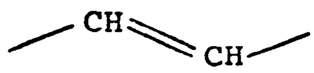 oder $-CH_2-CH_2-$Gruppe.

2. Verfahren zur Herstellung von Verbindungen der Formel I, dadurch gekennzeichnet, daß man

a) eine Verbindung der Formel II

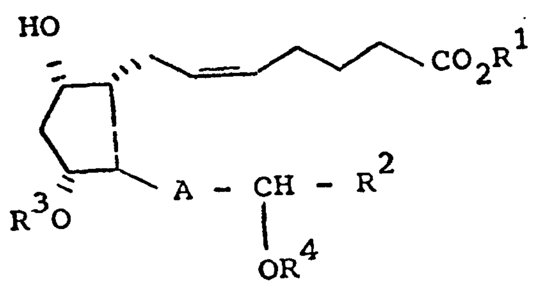

II

worin $R^1$, $R^2$ und A die zur Formel I angegebenen Bedeutungen haben und $R^3$ und $R^4$ gleich oder verschieden sein können und Wasserstoff oder eine leicht abspaltbare Schutzgruppe bedeuten, mit einem geeigneten elektrophilen Reagens umsetzt zu einer Verbindung der Formel III

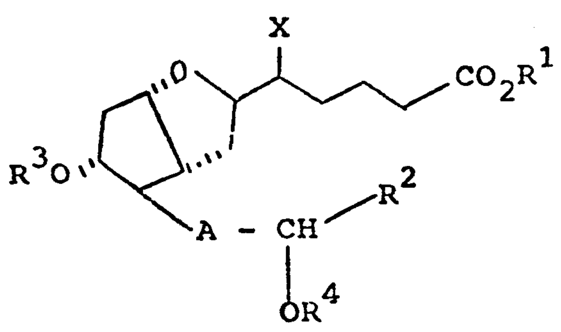

III

worin $R^1$, $R^2$ und A die zur Formel I, $R^3$ und $R^4$ die zur Formel II angegebenen Bedeutungen haben und X Chlor, Brom oder Jod bedeutet,

$a_1$) sofern in einer Verbindung der Formel III $R^3$ und/oder $R^4$ eine Schutzgruppe bedeutet, diese abspaltet, wobei eine Verbindung der Formel III erhalten wird, in welcher $R^1$, $R^2$ und A die zur Formel I und X die zur Formel III angegebenen Bedeutungen haben und $R^3$ und $R^4$ Wasserstoff bedeuten,

b) aus einer Verbindung der Formel III HX abspaltet, wobei eine Verbindung der Formel IV

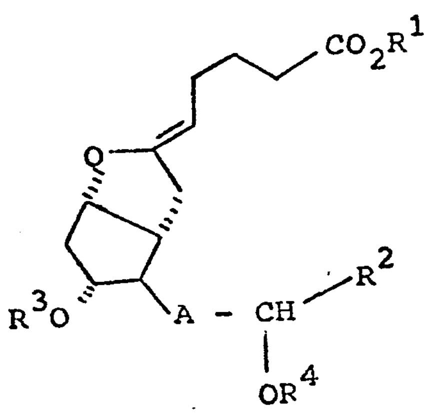

IV

erhalten wird, worin $R^1$, $R^2$ und A die zur Formel I und $R^3$ und $R^4$ die zur Formel II angegebenen Bedeutungen haben,

c) sofern in einer Verbindung der Formel IV $R^3$ und/oder $R^4$ nicht Wasserstoff, sondern eine Schutzgruppe bedeutet, diese unter geeigneten neutralen oder alkalischen Bedingungen abspaltet, wobei eine Verbindung der Formel I gebildet wird, worin $R^1$, $R^2$ und A die zur Formel I angegebenen Bedeutungen haben,

$c_1$) gegebenenfalls aus einer Verbindung der Formel III, sofern $R^3$ und/oder $R^4$ nicht Wasserstoff, sondern eine Schutzgruppe bedeutet, diese gleichzeitig mit HX abspaltet, wobei eine Verbindung der Formel I gebildet wird, worin $R^1$, $R^2$ und A die zur Formel I angegebenen Bedeutungen haben,

d) gegebenenfalls eine Verbindung der Formel I, worin $R^1$ Wasserstoff oder ein Kation bedeutet und A und $R^2$ die zur Formel I angegebenen Bedeutungen haben, zu einer Verbindung der Formel I verestert, worin $R^1$ ein Alkylrest mit der zur Formel I angegebenen Bedeutung ist und A und $R^2$ die zur Formel I angegebenen Bedeutungen haben,

e) gegebenenfalls eine Verbindung der Formel I, worin A und $R^2$ die zur Formel I angegebenen Bedeutungen haben und $R^1$ einen Alkylrest bedeutet, zu einer Verbindung der Formel I verseift, worin A und $R^2$ die zur Formel I angegebenen Bedeutungen haben und $R^1$ Wasserstoff oder ein physiologisch verträgliches Kation bedeutet,

f) gegebenenfalls in einer Verbindung der Formel I, worin A und $R^2$ die zur Formel I angegebene Bedeutung haben und $R^1$ ein physiologisch verträgliches Metall-, $NH_4$-oder Ammoniumion bedeutet, das sich von einem primären, sekundären oder tertiären Amin ableitet, das Kation $R^1$ gegen ein anderes austauscht.

3. Verbindungen der Formel III

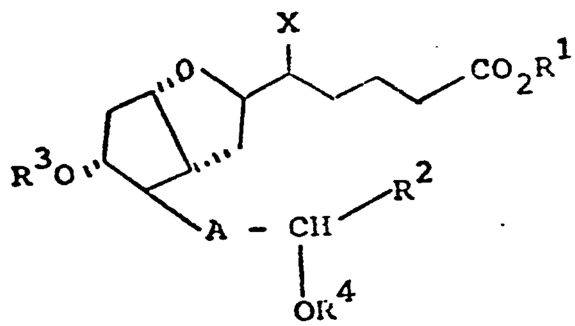

III

worin $R^1$, $R^2$ und A die zur Formel I, $R^3$ und $R^4$ die zur Formel II angegebenen Bedeutungen haben und X Chlor, Brom oder Jod bedeutet.

4. Verbindungen der Formel IV

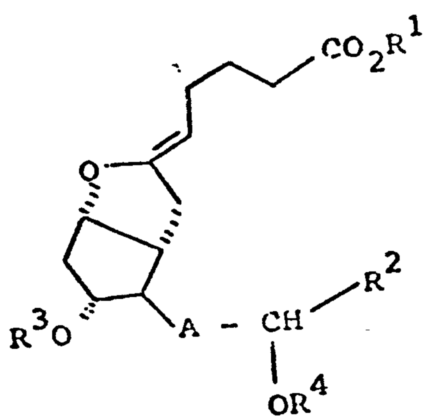

IV

worin $R^1$, $R^2$ und A die zur Formel I und $R^3$ und $R^4$ die zur Formel II angegebenen Bedeutungen haben.

5. Verbindungen der Formel I zur Verwendung als Arzneimittel.